# EUROPEAN PATENT APPLICATION

(11) **EP 1 498 128 A1**
(43) Date of publication of application: **19.01.2005**
(21) Application number: 03717677.3
(22) Date of filing: 21.04.2003
(51) Int. Cl.: A61K 31/722, A61K 31/727, A61K 38/22, A61K 45/00, A61K 9/06, A61L 15/16, A61L 27/20, A61L 27/44, A61P 17/02

(54) **MEDICINAL COMPOSITIONS CONTAINING PHTOCROSSLINKABLE CHITOSAN DERIVATIVE**

(30) Priority: 23.04.2002 JP 2002120995
(71) Applicant: Netech Inc., Kawasaki-shi, Kanagawa 213-0012 (JP)
(72) Inventor: ISHIHARA, Masayuki, Tachikawa-shi, Tokyo 190-0033 (JP); YURA, Hirofumi, Kawasaki-shi, Kanagawa 213-0011 (JP); SAITO, Yoshio, Yokohama-shi, Kanagawa 236-0011 (JP); OBARA, Kiyohaya, Tokorozawa-shi, Saitama 359-1145 (JP); FUJITA, Masanori, Tokorozawa-shi, Saitama 359-0045 (JP)
(74) Representative: Poulin, Gérard
(86) International application number: PCT/JP2003/005070
(87) International publication number: WO 2003/090765

(57) **Abstract**

A medical composition is provided which is advantageous as a sealant for wound openings as well as have a function promoting healing of intractable wound or incision wound, and which accelerate tissue regeneration but does not cause any side effect such as canceration. The present invention relates to a medical composition comprising a photo-crosslinkable chitosan derivative and a wound healing promoter. The photo-crosslinkable chitosan derivative is preferably a polymer obtainable by incorporating a carbohydrate chain containing a reducing terminal to at least one portion of amino groups of chitosan back bone and incorporating a photoreactive group to at least another part of the amino groups. The wound healing promoter is preferably a growth factor.

## Description

### TECHNICAL FIELD

The present invention relates to a medical composition comprising a photo-crosslinkable chitosan derivative and a wound healing promoter, more particularly to a composition combining adhesive activity and wound healing promoting activity, capable of adhering to an intractable skin disease such as a diabetic skin ulcer or a pressure sore, or a wound, and healing it by appropriately sustained-releasing the wound healing promoter; and a method of manufacturing said composition having said photo-crosslinkable chitosan derivative as the base agent; and applications of said composition.

### BACKGROUND ART

Wounds in skin and the like are mainly categorized into open wounds in which normal skin and mucosa tissues are separated and opened, burns caused by heat or radiation, ulcers (circumscribed tissue defect), complexed wounds thereof, and the like. In particular, for a severe wound having a wide area and a deep depth, treatment that prevents exogenous inflammation due to infections and promotes the derivation of granulation tissues is essential.

For the treatment of such wounds, wound dressings such as gauze have long been used. However, since wound dressings themselves have no healing effects, innovations have been made to improve healing effects by concurrently using drugs such as antibiotics. Recently, due to advances in materials chemistry, transparent adhesive films and gelatinous hydrogels that prevent water intrusion while having gas permeability, and further, artificial skin-like dressings utilizing biological membranes from animals or the like have come to be used. However, nothing has yet been provided that contains both protection functions such as wound protection and defense against infection, and healing functions that promote healing at the same time.

In particular, in intractable wounds such as pressure sores or skin ulcers derived from diabetes, since blood circulation in defective tissues is inhibited, regeneration of the defective tissues is significantly delayed. In the present state of affairs, curative drugs, dressings and the like that are effective for the promotion of healing have not been provided, so there is a risk that severe exogenous infections may develop concomitantly during the long period required for healing, and in cases where infections are concomitantly developed, in order to treat them, there is no choice but to conduct surgical procedures for which there is a large burden on the patient, such as the root-and-branch excision of necrotic tissues.

For the treatment of such intractable wounds, the use of regenerated tissues obtained by culturing tissue cells *in vitro* has been considered, but problems such as convenience, cost, time, safety and the like are still left. Recently, research on treatment methods using genes that promote vascularization in order to induce tissue regeneration has been advanced, but many problems have yet to be resolved, such as the methodology for gene transfer and whether or not proteins produced by transgenes have clinical effects.

Further, other than the abovementioned kinds of wounds, there are surgical excision wounds from the treatment of organs such as the stomach or the lungs, and for such wounds, dressings having a high adhesiveness are used as sealants for preventing the leakage of air or body fluids in the anastomotic parts of tissues. For example, biologically-derived protein and cyanoacrylate-based synthetic adhesives, and further, human-derived plasma formulations such as blood coagulation factors are used. However, in the present state of affairs, their adhesiveness, safety, biodegradability and the like are not sufficient. In particular, it is important to induce tissue regeneration in incisions and having proactive healing promoting effects, in order to contribute largely to the prognosis of patients, but a practical sealant having such functions has yet to be provided.

Meanwhile, it is known that growth factors (GF) control the propagation and differentiation of cells, and contribute to tissue regeneration. In particular, research on the fibroblast growth factors FGF-1 and FGF-2 has been advancing, and it has become apparent that these control the propagation, migration, differentiation, life span and the like of cells, and contribute to fetal development, vascularization, bone formation, nerve formation, and wound repair. However, it is difficult to maintain and store the activity of GF, and generally, GF interacts with heparin molecules in the proteoglycan constituting the extracellular matrix, thereby protection against deteriorization from oxidation and function adjustment is done. Further, when concentrated GF acts at one time, there is a risk that cancer-like elements might be elicited, so it must be used carefully. That is, though it has been expected that the tissue regeneration function of GF could be utilized for wound treatment, its actual clinical application has been hindered by the problems of GF itself, such as high diffusivity and rapid functional deterioration.

The inventors of the present invention have been developing a therapeutic agent for wounds utilizing a polysaccharide, chitosan (refer to WO00/27889). Chitosan had been known as a material combining both wound healing effects and antibacterial activity, but since the control of water solubility and gelation was difficult, it was difficult to use conventionally as a multipurpose and functional adhesive or dressing. We have improved by chemical modification the physical characteristics of chitosan, which solubilized only in acid regions, so that it is readily soluble in neutral and alkaline regions, and at the same time, introduced a photoreactive group that can functionally makes intermolecular crosslinking. The photo-crosslinkable chitosan derivative thereby obtained forms a viscous aqueous solution in physiological pH, and said aqueous solution becomes a contact type insoluble gel under irradiation by light. Therefore, it has become evident that an aqueous solution of the photo-crosslinkable chitosan derivative which was developed, can be applied to any affected area, and can immediately produce an adhesive gel by photoreaction at said area, and therefore may be used effectively as an adhesive for wounded areas.

### SUMMARY OF THE INVENTION

It is an object of the present invention to provide a medical composition which promotes tissue regeneration and at the same time is free from the risk of causing side effects such as canceration, through adding the function of proactively promoting the healing of intractable wounds and surgical incision wounds to the composition containing the photo-crosslinkable chitosan derivative we developed that has the advantageous functions as a sealant for wounds such as (1) strong and quick adhesiveness, (2) healing characteristics, (3) safety, and (4) appropriate biodegradability.

Therefore, the present invention provides a medical composition characterized in that it contains the photo-crosslinkable chitosan derivative and a wound healing promoter.

In addition to possessing advantageous characteristics such as the foregoing (1) through (4) as a sealant (medical adhesive) for wound regions, this composition is prepared in physiological pH, so that wound healing promoters having physiological activity, such as GF, can be favorably incorporated without being denatured. The obtained composition can be applied to any wound region, and becomes an occlusive and insoluble gel matrix by merely irradiating said region with light. The crosslinked gel matrix obtained from said photo-crosslinkable chitosan derivative not only rigidly holds wound treatment promoters such as growth factors, but also sustained-releases the held GF and the like at an appropriate rate. Due to this appropriate sustained-release characteristic, vascularization induction effects can be realized over a long period without canceration of tissues by high doses of growth factor. As a result, healing of intractable wounds and incision wounds can be promoted.

Further, the present invention also provides a composition concurrently holding glycosaminoglycans such as heparin, which are important for functional control of GF. Differently from the conventional basic chitosan, in which controlling the formation of a polyion complex with acidic heparins is difficult, the photo-crosslinkable chitosan derivative used in the present invention can easily hold heparins, and can improve GF function at the affected area by interaction with heparin.

Therefore, the present invention also provides a crosslinked chitosan matrix manufactured by irradiating a composition containing the photo-crosslinkable chitosan derivative and the wound healing promoter, and containing glycosaminoglycans if desired. This matrix can be used as a drug releasing body for releasing wound healing promoters such as growth factors and heparins held therein at an appropriate rate. In particular, the matrix holding the growth factor can be also used as a cell culture medium for tissue regeneration, due to the activity of the released growth factor.

### BRIEF DESCRIPTION OF THE DRAWINGS

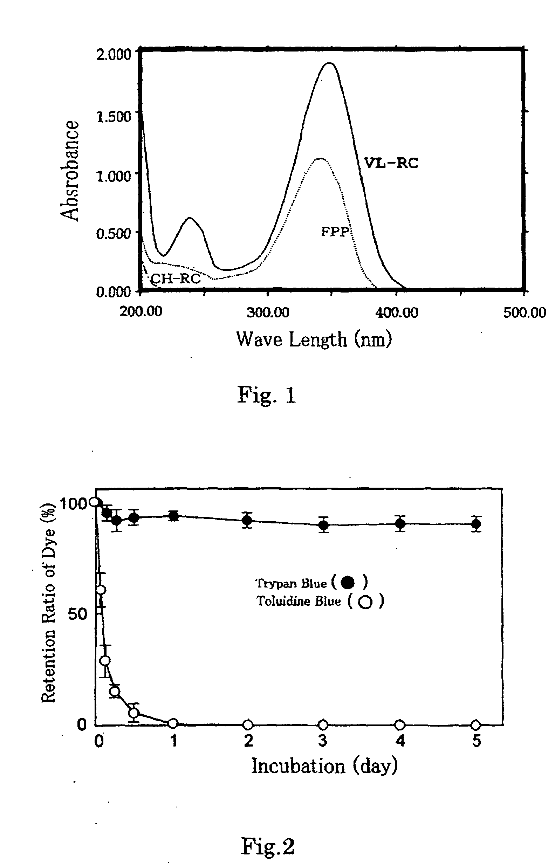

Fig. 1 is a graph showing a light absorption spectrum for aqueous solutions of lactose introduced chitosan derivative (CH-LA), visible light reactive compound (FPP) and visible light crosslinkable type chitosan derivative (VL-RC).
Fig. 2 is a graph showing *in vitro* release of low-molecular dyes (trypan blue and toluidine blue) from the crosslinked chitosan matrix of the present invention to PBS.

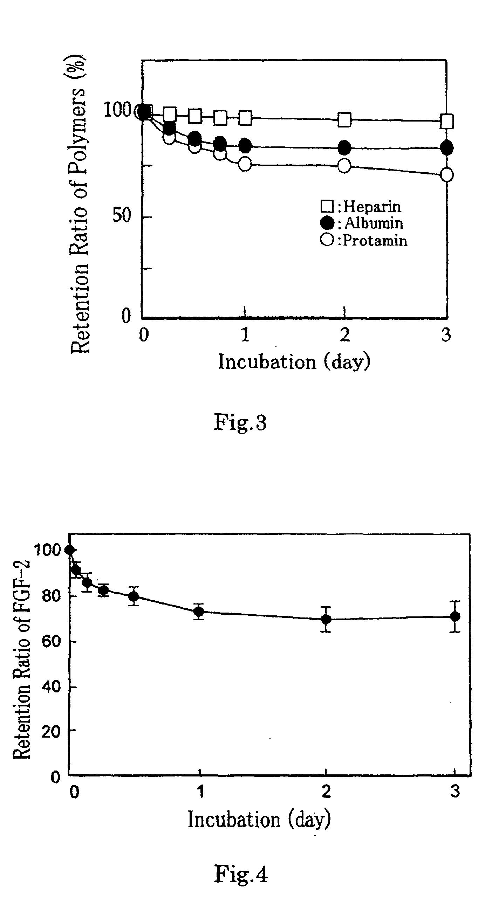

Fig. 3 is a graph showing *in vitro* release of polymer substances (polysaccharides and proteins) from the crosslinked chitosan matrix of the present invention to PBS.
Fig. 4 is a graph showing *in vitro* release of polymer substances (FGF-2) from the crosslinked chitosan matrix of the present invention to PBS.

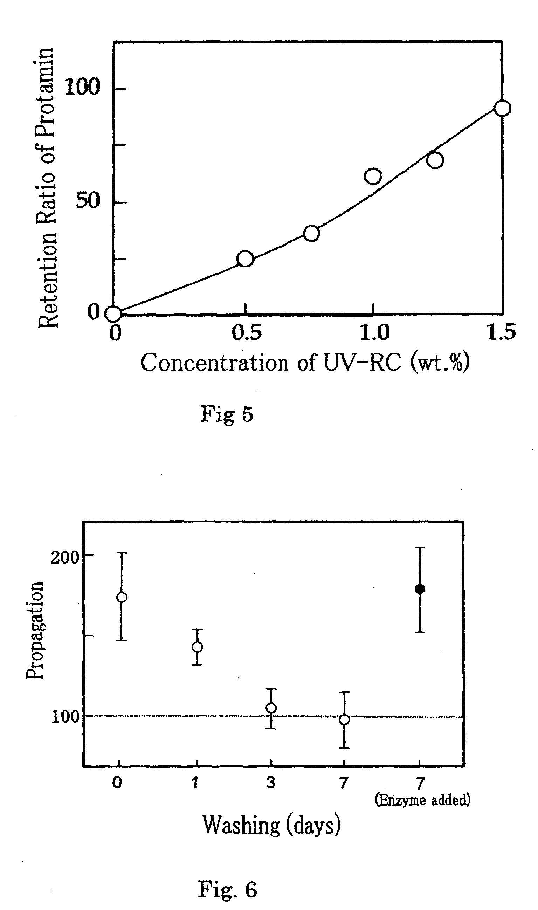

Fig. 5 is a graph showing the relationship between the molecular density of crosslinked chitosan matrix (chitosan derivative concentration) and retention ratio of substances held therein.
Fig. 6 is a graph showing the relationship between the growth of HUVEC on the crosslinked chitosan matrix of the present invention and release of FGF-2 from the matrix.

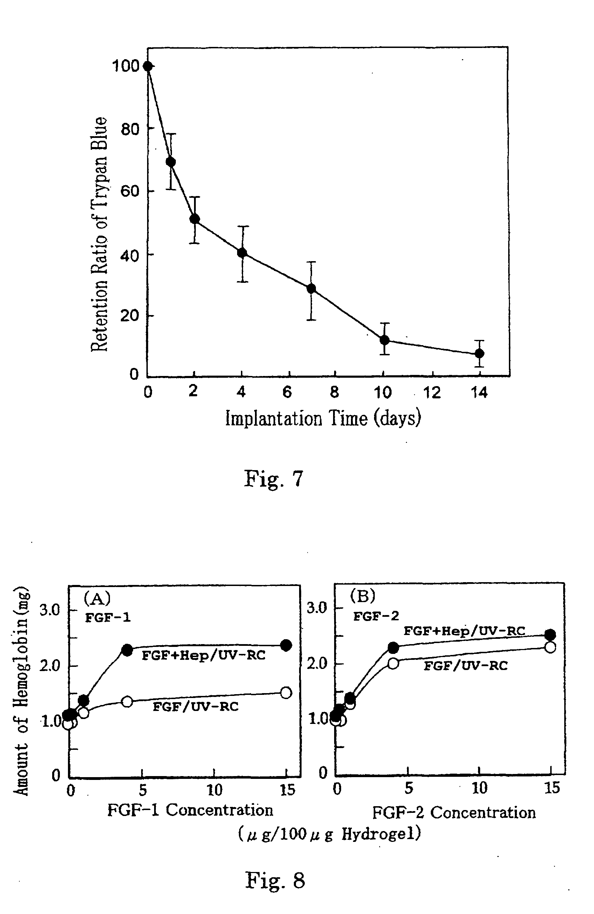

Fig. 7 is a graph showing *in vivo* release of low-molecular dye (trypan blue) from the crosslinked chitosan matrix of the present invention.
Fig. 8 is a graph showing angiogenesis effect by sustained release of growth factor (FGF) from the crosslinked chitosan matrix of the present invention. This demonstrates the relationship between the held FGF concentration and the amount of hemoglobin. ○ indicates FGF-carrying chitosan matrix, and • indicates matrix further containing heparin.

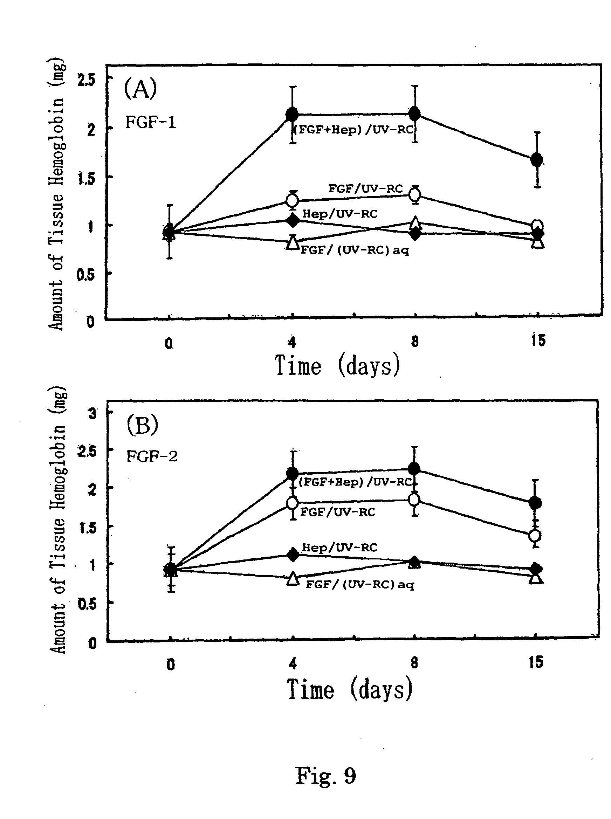

Fig. 9 is a graph showing angiogenesis effect by sustained release of growth factor (FGF) from the crosslinked chitosan matrix of the present invention. This demonstrates the time-dependent change of the amount of hemoglobin. ○ indicates FGF-carrying chitosan matrix, ● indicates matrix further containing heparin, ◆ indicates a comparative example carrying only heparin, and Δ indicates aqueous solution of UV-RC supplemented with FGF.

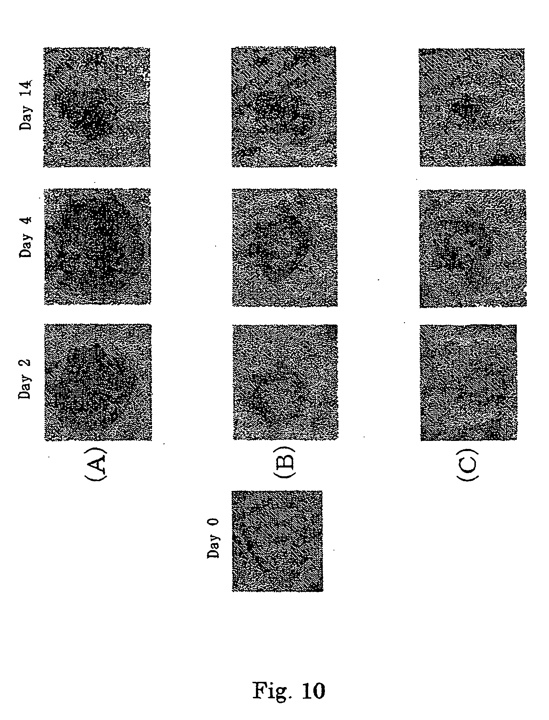

Fig. 10 is a photograph showing healing effect of the crosslinked chitosan matrix of the present invention in a wound model formed in db/db mouse. (A) indicates untreated control, (B) indicates a wound coated with crosslinked chitosan matrix without FGF, and (C) indicates a wound coated with crosslinked chitosan matrix of the present invention further containing FGF.

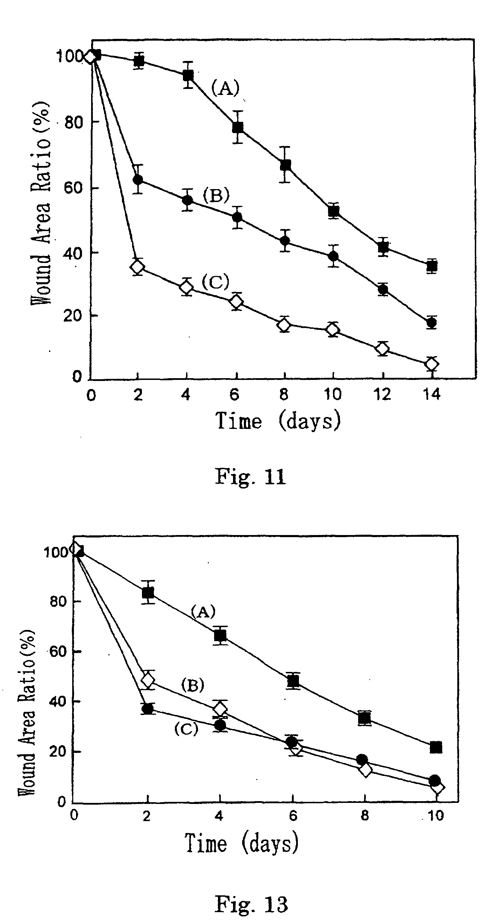

Fig. 11 is a graph showing healing effect of the crosslinked chitosan matrix of the present invention in a wound model formed in db/db mouse. (A) indicates untreated control, (B) indicates a wound coated with crosslinked chitosan matrix without FGF, and (C) indicates a wound coated with crosslinked chitosan matrix of the present invention further containing FGF.

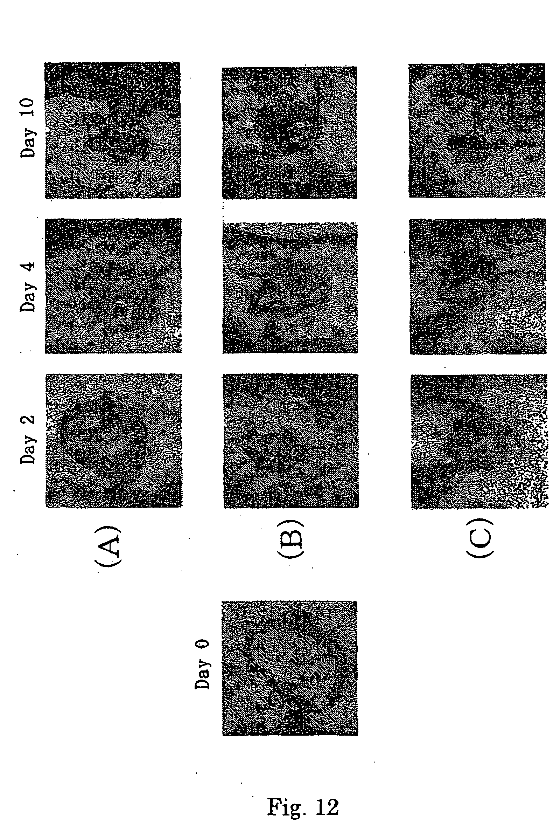

Fig. 12 is a photograph showing healing effect of the crosslinked chitosan matrix of the present invention in a wound model formed in db/+ mouse. (A) indicates untreated control, (B) indicates a wound coated with crosslinked chitosan matrix without FGF, and (C) indicates a wound coated with crosslinked chitosan matrix of the present invention further containing FGF.

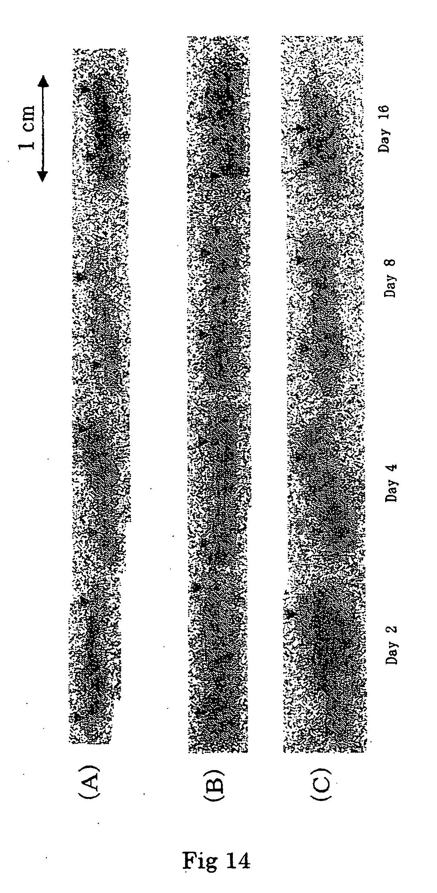

Fig. 13 is a graph showing healing effect of the crosslinked chitosan matrix of the present invention in a wound model formed in db/+ mouse. (A) indicates untreated control, (B) indicates a wound coated with crosslinked chitosan matrix without FGF, and (C) indicates a wound coated with crosslinked chitosan matrix of the present invention further containing FGF.
Fig. 14 is a photograph showing time-dependent change of wound area in a wound model formed in db/db mouse. (A) indicates untreated control, (B) indicates a wound coated with crosslinked chitosan matrix without FGF, and (C) indicates a wound coated with crosslinked chitosan matrix of the present invention further containing FGF.

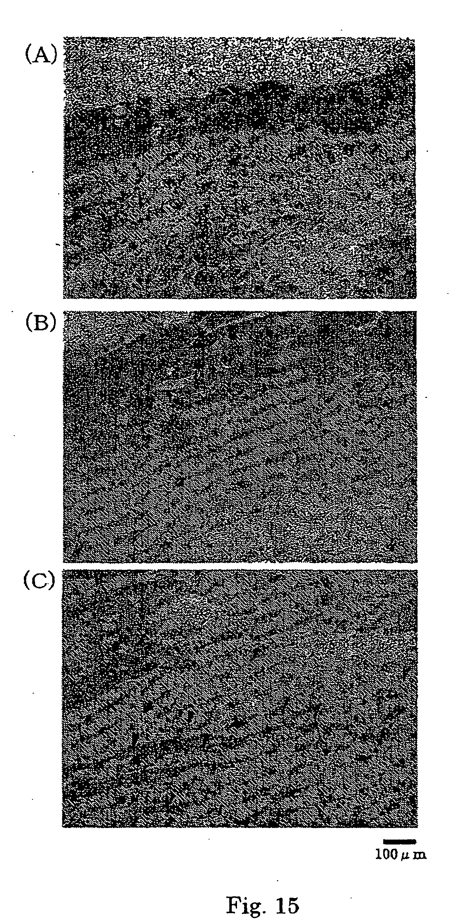

Fig. 15 is a microscopic photograph of a sample obtained by staining with HE the cross-section of the wound at fourth day in a wound model formed in db/db mouse. (A) indicates untreated control, (B) indicates a wound coated with crosslinked chitosan matrix without FGF, and (C) indicates a wound coated with crosslinked chitosan matrix of the present invention further containing FGF.
Fig. 15 is a microscopic photograph of a sample obtained by staining with CD34 the cross-section of the wound at fourth day in a wound model formed in db/db mouse. (A) indicates untreated control, (B) indicates a wound coated with crosslinked chitosan matrix of the present invention further containing FGF.

### BEST MODE FOR CARRYUNG OUT THE INVENTION

The photo-crosslinkable chitosan derivative usable in the medical composition of the present invention preferably has a structure in which a photo-crosslinkable group and a carbohydrate chain are introduced into a polymer back bone, which is generally called as chitin/chitosan. In particular, those formed by incorporating a carbohydrate having reducing terminals and a photo-reactive functional group to at least a portion of the 2-position amino groups in the glucosamin units constituting an at least partially deacetylated chitin/chitosan are preferable.

Normally, chitin/chitosans are deacetylated acid-soluble fractions obtained by alkali processing chitin (poly-N-acetylglucosamins) originated from crab shells, and generally have the constituent units expressed by the following formulas (1) and (2).

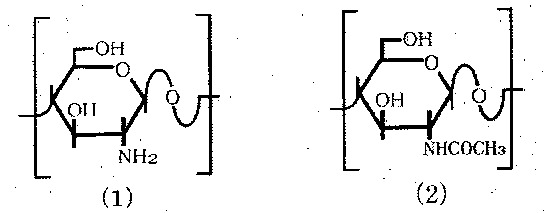

Among chitin/chitosans, some persons call those having a low degree of deacetylation (normally less than 40%) as "chitins" and those having a high degree of deacetylation (normally 40% or more) as "chitosans", but henceforth in the present specification, all chitin/chitosans which are at least partially deacetylated shall be referred to collectively as "chitosans". Additionally, in the present invention, chitosans are not limited to those of natural origin, and may be chemically modified carbohydrate chains having similar structures synthesized chemically or by genetic engineering.

Here, "degree of deacetylation" refers to the proportion of acetylamino groups in the 2-position of the carbohydrate units constituting the chitosan (or poly-N-acetylglucosamin), which have been converted to free amino groups by deacetylation. In the present specification, the degree of deacetylation is measured by means of the "colloidal titration method" described in "Health Foods Standard and Criterion (No. 4)", Japan Health Food and Nutrition Food Association (1996), p. 55.

The chitosan derivative of the present invention has been functionalized by further chemically modifying the chitosan, and the chitosan used as the raw material should preferably have a degree of deacetylation of at least 40%, preferably 60-100%, more preferably 65-95%. A chitosan having a 100% degree of acetylation consists entirely of the constituent units of the above-given formula (1), and does not include the constituent units of formula (2).

Additionally, there are no particular restrictions on the molecular weight of the chitosan, and this can be changed of a wide range depending on the projected use of the chitosan derivative, but in general, the number-average molecular weight should be in the range of 5,000-2,000,000, preferably 10,000-1,800,000, more preferably 40,000-1,500,000.

The chitosan derivatives suitable for the present invention are those formed by incorporating a carbohydrate having reducing terminals to at least a portion of the 2-position amino groups in the glucosamin units (1) constituting the above-described chitosan and a photo-reactive functional group to at least another portion of the 2-position amino groups. Details of such chitosan derivatives are described in WO00/27889.

The carbohydrates having reducing terminals to be incorporated to the chitosan derivatives include aldoses and ketoses, among which those having 20 or less constituent carbohydrate units, especially those with 1-7 units are preferably used. Specific examples include pentaoses and hexaoses such as glucose, fructose, galactose, fucose, mannose, arabinose, xylose, erythrose, hepturose and hexylose, amino carbohydrates such as glucosamin, N-acetylglucosamin and galacsamin; carbohydrate derivatives such as uronic acids and deoxysaccharides; di- and trisaccharides such as maltose, isomaltose, lactose, melibiose and maltotriose composed of carbohydrate chains combining the above-mentioned monosaccharides; and the various oligosaccharides, among which the neutral disaccharides such as maltose, lactose and melibiose are preferable.

While it is also possible to derive chitosans from organic compounds such as polyethers and polyhydric alcohols instead of the above-mentioned carbohydrates, it is preferable to use natural carbohydrate chains in consideration of biocompatibility.

The incorporation of the above-mentioned carbohydrates in the 2-position amino group of the glucosamin units of the chitosan of the above-given formula (1) can itself be performed using known methods. For example, methods of carboxylating the reducing terminal of a carbohydrate, then binding to the 2-position amino group by an amide bond (see, for example, Japanese Patent Application, First Publication No. H10-120705), or of aldehydating or carbonylating the reducing terminal of a carbohydrate, then binding to the 2-position amino group of a glucosamin unit by a reduction alkylation method by means of a Schiff base (see, for example, "Applications of Chitins and Chitosans", edited by Chitin/Chitosan Workshop, pp. 53-56, Feb. 20, 1990, published by Gihodo Shuppan KK).

The carbohydrate incorporated in the chitosan in the present invention is not limited to only one type, and it is possible to use a combination of 2 or more.

Specific examples of a carbohydrate side chain constituting the chitosan derivative of the present invention include the following, but there is no restriction to these.
(i) Carbohydrate derived from lactose: 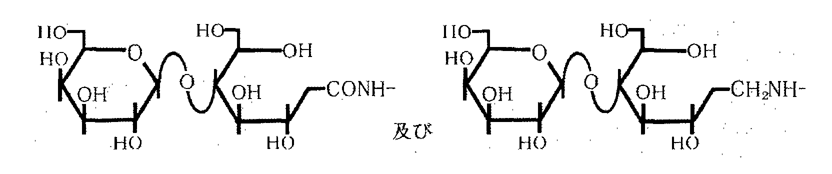
(ii) Carbohydrate derived from maltose: 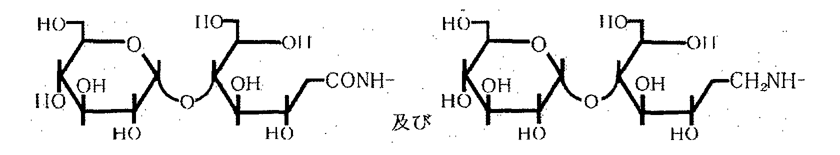
(iii) Carbohydrate derived from melibiose: 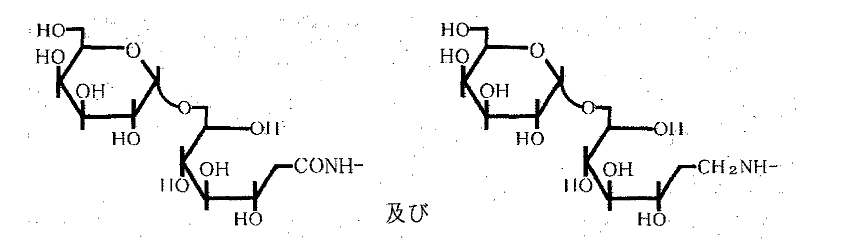
(iv) Carbohydrate derived from cellobiose: 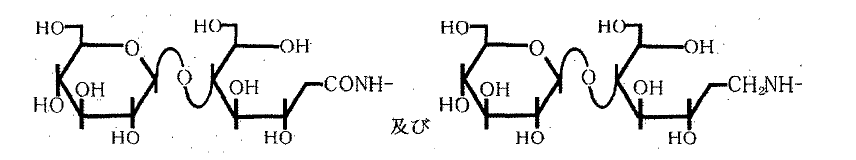
(v) Carbohydrate derived from laminalibiose: 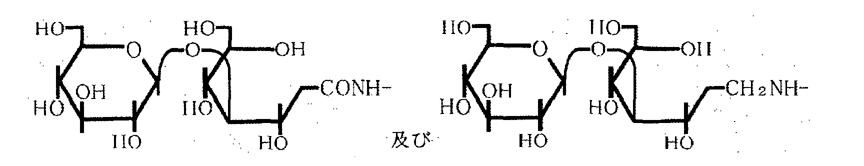
(vi) Carbohydrate derived from mannobiose: 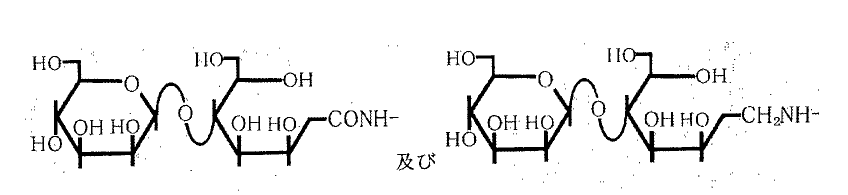
(vii) Carbohydrate derived from N-acetylchitobiose:

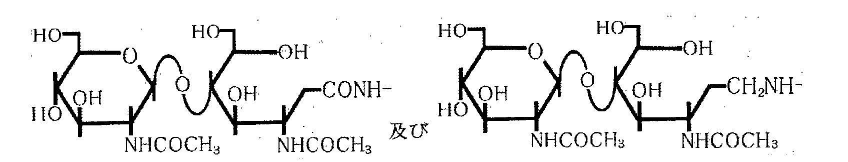

Of the carbohydrate side chains given in the above (i)-(vii), those on the left side represent residual groups incorporated by means of condensation between a carboxyl group on the carbohydrate and a 2-position amino group on the chitosan, while those on the right side represent residual groups bound by a Schiff base.

While the degree of substitution of 2-position amino groups in the glucosamin units of chitosan by carbohydrate side chains can be changed depending on the physical properties desired in the final chitosan derivative, the degree of substitution should generally be in the range of 0.1-80%, preferably 0.5-60%, more preferably 1-40%. Here, the "degree of substitution" of the carbohydrate side chain is the level to which the amino groups in the 2-position of the carbohydrate units constituting the chitosans are substituted by carbohydrate side chains, and denote the proportion of substituted amino groups with respect to the total number of free amino groups and substituted amino groups at the 2-position of the carbohydrate units constituting the chitosans. In the present specification, the degree of substitution of carbohydrate side chains is measured by the "phenol -sulfuric acid method" wherein the characteristic color emission due to a reaction between carbohydrate chains and phenol in sulfuric acid is sensed by light absorption at 490 nm (see J. E. Hodge, B. T. Hofreiter, "Methods in Carbohydrate Chemistry", ed. by R. L. Whistler, M. L. Wolfrom, vol. 1, p. 388, Academic Press, New York (1962)).

The chitosan derivative of the present invention has a self-crosslinking property by photo-irradiation due to incorporating photo-reactive functional groups in the 2-position amino groups in the glucosamin units of the above-given formula (1) constituting the chitosan.

The photo-reactive functional groups used for chemical modification of the chitosans according to the present invention are groups which react with each other and/or amino groups or hydroxyl groups present in the chitosan upon irradiation by ultraviolet light including the near-ultraviolet region of 200-380 nm to form crosslinking bonds including, for example, those derivable from cyclic unsaturated compounds such as benzophenones, cinnamic acids, azides, diolefins and bis-anthracene, especially preferable being those having carbonylazide groups, sulfonylazide groups and aromatic azide groups.

The photo-reactive group may be a substitutional group which reacts by irradiation of visible light of about 400 to 500 nm. Such visible-light-reactive groups include, for example, formyl styryl group represented by the following formula and described in Journal of Polymer Science: Polymer Chemistry Edition, Vol. 20, 1419-1432 (1982). 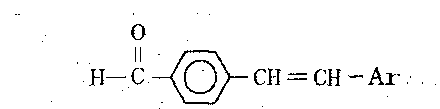 (In this formula, Ar denotes a heterocyclic ring such as pyridin, alkylpyridinium salt, quinolin, or alkylquinolinium salt.)

The incorporation of photo-reactive functional groups to the amino groups at the 2-position in the glucosamin units of the chitosans can itself be performed by known methods, for example, by a method of binding an azide compound having a carboxyl group to the 2-position amino group in the presence of a condensing agent (see Japanese Patent Application, First Publication No. H10-120705); or a method of reacting the azide compound with the 2-position amino group by means of an acid chloride group, an aldehyde group, an N-hydroxysuccinic acid imide ester group or an epoxy group (see "Applications of Chitins and Chitosans", edited by Chitin/Chitosan Workshop, pp. 53-5645-65, Feb. 20,1990, published by Gihodo Shuppan KK). In azide group crosslinking reactions, it has been conventionally held to be effective to use polyfunctional compounds such as bis-azides or above (see Japanese Patent Application, First Publication No. H9-103481), this is not necessary in the present invention, so that a chitosan derivative having adequate self-crosslinking ability can be obtained by incorporation of monoazide compounds.

Specific examples of a photo-reactive group forming the chitosan derivative of the present invention include, for example, those expressed by the following formulas (A) through (E). The group of formula (A) is derived from p-azidobenzoic acid, the group of formula (B) is derived from p-azidobenzaldehyde, the group of formula (C) is derived from p-benzoylbenzoic acid, the group of formula (D) is derived from cinnamic acid, and the group of formula (E) is derived from 1-methyl-4-[2-formylphenyl]ethenyl]pyridinium. 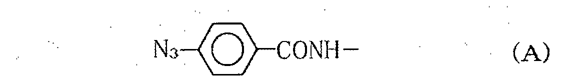 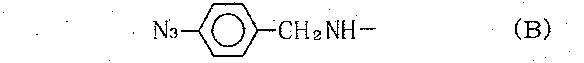 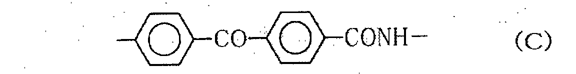 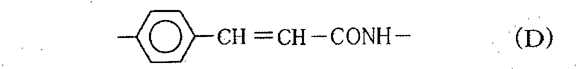 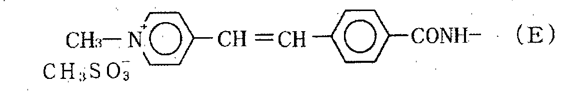 While the degree of substitution of these photo-reactive functional groups can be changed according to the degree of gelification (insolubility) due to the crosslinking reaction desired in the final chitosan derivative, but it is preferable for the degree of substitution of the photo-reactive functional groups to be within the range of 0.1-80%, preferably 0.5-50%, more preferably 1-30%. Here, the "degree of substitution" of the photo-reactive functional groups is the degree of substitution of the 2-position amino groups of the carbohydrate units forming the chitosans with photo-reactive functional groups, and is the proportion of substituted amino groups with respect to the total number of free amino groups and substituted amino groups at the 2-position of the carbohydrate units forming the chitosans. In the present specification, the degree of substitution of photo-reactive functional groups such as azide groups can be determined based on calibration curves obtained from characteristic absorption at 270 nm for 4-azidobenzoic acid.

The degree of substitution of the total of carbohydrate side chains and photo-reactive functional groups in the chitosan derivatives of the present invention is not particularly restricted, and may vary over a considerable range, but is usually in the range of 0.2-80%, preferably 1.5-65%, more preferably 3-50%.

Additionally, according to the present invention, a hydrogel with considerably improved water retention ability can be obtained by incorporating an amphipathic group to at least a portion of the 3- or 6-position hydroxyl groups in the carbohydrate units of formulas (1) and (2), and the amino groups in the 2-position of the carbohydrate units of formula (1) constituting the chitosan. These amphipathic groups are groups having a hydrophobic block comprising a hydrophobic group and a hydrophilic block comprising a hydrophilic group, and often have a surfactant function. Among these those in which the molecular weight ratio between the hydrophobic blocks (X) and the hydrophilic blocks (Y) is X : Y = 1 : 5 to 5 : 1 are preferably used, and non-ionic groups without dissociated ionic groups are more preferably used. In particular, those composed of a hydrophobic alkyl block and a hydrophilic polyoxyalkylene block and with a molecular weight of at least 90 are preferable, a polyoxyalkylene alkyl ether of 500-10,000 being more preferable. While a polyether not having a hydrophobic block may be used, a polyoxyalkylene alkyl ether is preferable for having both a hydrophobic block and a hydrophilic block in consideration of the improvement to the water retaining ability.

The incorporation of these amphipathic groups to the chitosan can be performed, for example, by a method of incorporating a compound having groups capable of reacting with amino groups to form covalent bonds, such as aldehyde groups or epoxy groups to a terminal portion of either the hydrophilic block or hydrophobic block of the amphipathic group, then reacting with the 2-position amino group of the glucosamin of the chitosan, a method of inducing a reaction between a polyoxyalkylene alkyl ether derivative having a carboxyl group with the chitosan in the presence of a condensing agent, or a method of inducing a reaction between a polyoxyalkylene alkyl ether derivative having an acid chloride group with a hydroxyl group or amino group in the chitosan.

For example, when incorporating a polyoxyalkylene alkyl ether group with an epoxy group on its terminal into an amino group in the chitosan, the amphipathic group is expressed by the following formula (a), and when incorporating a polyoxyalkylene alkyl ether group with an aldehyde group on its terminal into an amino group of the chitosan, the amphipathic group is expressed by the following formula (b). Additionally, when binding a polyoxyalkylene alkyl ether group with an acid chloride group on its terminal to the 3- or 6-position hydroxyl group of the chitosan, the amphipathic groups are expressed by the following formula (c). In the below formulas (a)-(c), n and m are repeating units numbering 1 or more.

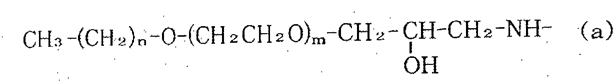

CH₃-(CH₂)ₙ-O-(CH₂CH₂O)ₘ-CH₂- CONH- (b)

CH₃-(CH₂)ₙ-O-(CH₂CH₂O)ₘ-CH₂-CO-CH₂- (c)

The degree of incorporation of amphipathic groups in the chitosan derivatives of the present invention is not particularly restricted, but should be within the range normally of 5-70%, preferably 15-55% based on the change in weight of the chitosan derivative after incorporation.

As described in detail above, in the photo-crosslinkable chitosan derivative used for the medical composition of the present invention, carbohydrates having a reducing terminus and a photoreactive group may be introduced into the chitosan backbone structure, and amphipathic groups can be introduced therein as desired. By introducing the carbohydrate, the chitosan derivative becomes well soluble in neutral regions, can be made into a solution by a physiological buffer or a culture media, and can be mixed without losing the activity of drugs, such as proteins, that may get denatured by acid or alkali. Further, by introducing the photoreactive group, an insoluble gel body may be formed immediately by light irradiation after application to an appropriate region, which adheres to tissues, and a wound healing promoter may be enclosed therein and sustained-released later.

As the polymer constituting the backbone structure of the photo-crosslinkable chitosan derivative of the present invention, instead of chitosan, polysaccharides such as hyaluronic acid, proteins such as collagen, and other synthetic polymers and the like can be used, but carbohydrates capable of sealing wounds and tissues, having drug holding characteristics and appropriate biodegradability, and having an ability to conduct controlled release of a drug at a speed which is not too fast are most suitable. Among these, chitosan, which itself has wound healing characteristics and antibiotic action, or carbohydrates such as hyaluronic acid are preferable, and chitosan is more preferable in view of the supply of raw materials and cost.

Further, it is possible to introduce a group having chemically crosslinkable characteristics instead of the photoreactive group. The introduced group is preferably capable of rapid intramolecular crosslinking and easy switching thereof. Since the photoreactive group has the characteristics of easy switching, high reactivity, and few unreacted active sites remain, the photoreactive group can be suitably used. Further, chitosan, having an amino group at the second position, is also advantageous for the introduction reaction of the photoreactive group, and therefore, they are suitably used.

The medical composition of the present invention contains at least one wound healing promoter in addition to the foregoing photo-crosslinkable chitosan derivative. The wound healing promoter in the present invention can be a substance capable of promoting healing effects in the wound region to which said composition is applied in every sense. Examples thereof include drugs having wound healing effects described in "Drug Directory Fifth Edition" (Pharmacists Association of Osaka Prefectural Hospital, Yakugyojiho Co., 1992) and its addenda edition (1992). For example, antibacterial agents and antibiotics for promoting healing by preventing infections and inflammation at wound regions, or acesodynes and anesthetic agents to alleviate pains caused by wounds are also included. In particular, when applied to an intractable skin disorder in which blood circulation is inhibited such as a pressure sore or a diabetic skin ulcer, substances inducing vascularization such as growth factors and angiogenesis factors are preferable. The growth factor used here is not particularly limited as long as the growth factor induces vascularization and has a function to promote granulation. For example, FGF-1, FGF-2, HB-EGF, HGF, VEGF₁₆₅, and HGF can be cited. Further, since the sustained-drug-releasing body of the present invention has extremely high tissue adhering characteristics, it is possible to conduct voluntary supply of the growth factor at the wound region by incorporating plasmids containing genes expressing the abovementioned growth factors.

Further, the medical composition of the present invention may further contain glycosaminoglycans such as heparin and heparan sulfate in addition to the foregoing photo-crosslinkable chitosan derivative and the wound healing promoter. For example, the growth factor is considered to adjust functions by interacting with heparin molecules in proteoglycan constituting the extracellular matrix existing in the vicinity of the wound tissues. Therefore, by letting the composition of the present invention contain glycosaminoglycans such as heparin, it is possible to give the composition of the present invention a function as a source of the glycosaminoglycans. These glycosaminoglycans can be simply mixed in the composition, or can be covalent-bound with the chitosan backbone structure as described in, for example, WO00/27889.

Further, the composition of the present invention may contain other medically acceptable additives. As examples, interleukin, leukemia inhibitor factor, interferon, TGF-β, erythropoietin, trombopoietin and the like are included. Further, other drugs known to induce apoptosis in mammals can be used. Examples of such drugs include TNF-α, TNF-β, CD30 ligand, and 4-1BB ligand. Further, chemotherapy drugs useful for treating cancers can be used. Examples of such chemotherapy drugs include an alkylating agent, folic acid antagonist, metabolic products of nucleic acids, antibiotics, pyrimidine analogs, 5-fluorouracil, cisplatin, purine nucleoside, amines, amino acid, triazole nucleoside, corticosteroids, and hormone drugs functioning to adjust or inhibit hormone action to tumors such as tamoxifen and onapristone.

The medical composition of the present invention can be prepared by dissolving the photo-crosslinkable chitosan derivative, the wound healing promoter, and other desired components into a solvent, preferably an aqueous medium in preferably neutral pH.

The composition prepared as above preferably has appropriate viscosity considering that such composition is applied to wound regions. For example, according to a commercially available rotary viscometer (for example, B type viscometer, manufactured by TOKIMEC Inc. (Tokyo, Japan)), when viscosity is set to about 100 to 10,000 cps (centipoise (mPa . s)), preferably about from 150 to 8,000, and more preferably from about 200 to 6,500 cps, application to a vertical wound or a tissue face becomes easy. Further, when viscosity is set to about from 250 to 5,000 cps, and preferably about from 300 to 2,000 cps, it becomes easy for application and free from runoff at any wound or tissue face, and holding time becomes an appropriate length, and enough time is allowed for subsequent procedures such as light irradiation. However, in application to a horizontal wound face, filling into a concave portion and the like, a composition having low viscosity of under about 300 cps, further under about 200 cps, even further under about 100 cps can be used. Further, for example, a composition having viscosity as low as, for example, pure water can be applied to regions other than a horizontal wound face by using a spray device and the like. In operative endoscopy through catheters or the like, appropriate viscosity can be adopted by considering fluidity in the tube, holding characteristics at the incised wound due to endoscopic operation, and the like.

That is, content of the photo-crosslinkable chitosan derivative in the medical composition of the present invention is set to at least 3 mg/ml, preferably at least 5 mg/ml, more preferably at least 7.5 mg/ml, much more preferably at least 10 mg/ml, and even much more preferably at least 15 mg/ml in order to secure holding characteristics at applied regions and securely hold the drugs contained in the matrix after crosslinking.

Content of the wound healing promoter is determined as appropriate in order to sustained-release the quantity required in the applied region. For example, content of the growth factor in the case of application to a skin wound region is set to about 1 to 1,000 µg/ml, preferably about 5 to 500 µg/ml, and more preferably about 10 to 200 µg/ml.

Although depending on the amount of the growth factor concurrently contained, content of glycosaminoglycan such as heparin is set to preferably about 10 to 5,000 µg/ml, more preferably about 50 to 1,000µg/mL and much more preferably about 100 to 500 µg/ml. Contents of other possible components are set to the degree generally used in the medical field, and their most suitable contents can be easily determined by those skilled in the art.

The medical composition of the present invention contains a photo-crosslinkable chitosan derivative. Therefore, by irradiating the medical composition of the present invention with light having a given strength (ultraviolet light, visible light and the like) for a predetermined time, crosslinking occurs in a short time to form an insoluble matrix. The formed crosslinked chitosan matrix holds additives such as wound healing promoter therein, and can sustained-release these additives at an appropriate rate.

Conditions for crosslinking by light vary according to the types and degree of substitution of the photoreactive groups introduced into the photo-crosslinkable chitosan derivative to be used, amounts of the chitosan derivative contained in the composition and amounts of the drug to be held, amounts of the composition to be used and desirable sustained-release rates and the like. In general, when about 100 µl of a composition containing at least about 7.5 mg/ml of the photo-crosslinkable chitosan derivative is used, light irradiation from a light source provided at about 2 cm from the composition is conducted for about 0.01 to 300 sec, preferably about 0.05 to 120 sec, and more preferably about 0.1 to 60 sec, and thereby about 100% crosslinking degree of the photoreactive group can be obtained.

By light irradiation, the photo-crosslinkable chitosan derivative crosslinks and an insoluble matrix is formed. The crosslinked chitosan matrix formed contains inside wound healing promoters such as growth factors and other additives such as heparin. These drugs may be initially released to some extent due to diffusion characteristics of the drug itself. However, most of the drug is rigidly held inside the crosslinked chitosan matrix. After that, when the crosslinked chitosan matrix is biodegraded, the held drug is sustained-released at an appropriate rate.

The crosslinking reaction degree of the photoreactive group of the chitosan matrix is not particularly limited. In general, it is considered that there is a trend that when the crosslinking reaction degree is high, initial release of the drug is small and suitable function as a drug releasing body can be obtained. Therefore, the crosslinked chitosan matrix of the present invention has a crosslinking reaction degree of at least 30, preferably from 40 to 100%, more preferably from 50 to 100%, much more preferably from 60 to 100%, and even much more preferably from 70 to 100%. Here, "crosslinking reaction degree (or crosslinking degree)" in the present invention represents the ratio of photoreactive groups bound with other groups and the like, among the photoreactive groups existing in the photo-crosslinkable chitosan derivative.

As a more specific usage form, the medical composition of the present invention is applied to tissues having, for example, a skin ulcer, and irradiated with light. Since the composition has appropriate viscosity, the composition is held without running off from the damaged region, rapidly becomes an adhesive and insoluble gel body (matrix) due to intermolecular crosslinking by light irradiation, seals the damaged region and concurrently sustained-releases the contained drug, and accelerates protection and healing of the wound. Further, in stomach walls, cancer tissues and the like, by containing the drug to treat them, treatment effects can be derived as a coating for the stomach ulcer and tumor tissues.

Therefore, the crosslinked chitosan matrix formed by irradiating the medical composition of the present invention with light constructs a very superior sustained-drug-releasing body, which is particularly suitable for healing wounds.

It is astonishing that the basic growth factor (FGF-2) conventionally considered not able to be held within the basic chitosan backbone structure has been held and sustained-released by the matrix formed from the photo-crosslinkable chitosan derivative of the present invention to a degree equal to or more than the acidic growth factor (FGF-1).

Further, when an amphipathic group such as polyoxyalkylene alkylether is further introduced into the photocrosslinkable chitosan derivative, the crosslinked chitosan matrix, after light irradiation, becomes able to rapidly absorb lots of moisture close to 100 times its own weight. In this case, the crosslinked chitosan matrix can absorb bleeding from the wound region or the surgery region to accelerate hemostasis.

Further, the crosslinked chitosan matrix of the present invention has the characteristics that the cell growth factor is held therein, and the cell growth factor is gradually released. Therefore, the crosslinked chitosan matrix of the present invention can also be used as a cell culture medium material for tissue regeneration. For example, the medical composition of the present invention is applied to the surface of the cell culture plate or the like, light irradiation is conducted, and a membranous crosslinked chitosan matrix is formed. When the target cell culture medium is arranged on the covered part of said cell culture plate and incubation is conducted, growth of said cells is stimulated. Therefore, in the present invention, the foregoing crosslinked chitosan matrix, specifically the matrix containing the growth factor can be effectively used as a cell culture medium material for tissue regeneration.

The composition of the present invention is generally provided as a viscous aqueous solution as described above. However, for example, the composition of the present invention can be provided as a solid (for example powder) obtained by freeze-drying the aqueous solution. The solid composition can be promptly used by being dissolved or swollen in an aqueous medium.

### Examples

Detailed descriptions of the present invention will be hereinafter given by using concrete examples. However, these concrete examples do not limit the scope of the present invention.

### (Synthesis Example 1)

### Synthesis of photo (ultraviolet) crosslinking chitosan derivative (UV-RC)

UV-RC wherein an ultraviolet reactive group and a carbohydrate chain were introduced into a chitosan backbone structure was synthesized in accordance with the method described in WO00/27889. More specifically, azide (p-azide benzoate) and lactose (lactobionic acid) were introduced by condensation reaction into an amino group of crab-derived chitosan having 800 to 1,000 kDa of molecular weight and 80% of deacetylation degree (available from Yaizu Suisan Industry Co., Ltd.). It was confirmed that the resultant was soluble in neutral pH due to introduction of lactose, and substitution degrees of p-azide benzoate and lactobionic acid were about 2.5% and 2.0% respectively.

Further, when chitosan materials derived from shrimp shell and a chitosan material derived from cuttlefish cartilage were used, similar derivatives could be synthesized.

### (Synthesis Example 2)

### Synthesis of photo (visible light) crosslinking chitosan derivative (VL-RC)

Methyl-4-[2-(4-formylphenyl)ethenyl] pyridine methosulfonate (FPP) expressed by the following formula was synthesized in accordance with the method described in Journal of Polymer Science: Polymer Chemistry Edition, Vol. 20, 1419-1432 (1982).

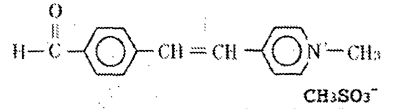

More specifically, under the condition of cooling with ice, a solution of γ-picolline (3.07 g, 33 mmol) in methanol (8.3 ml) was added to dimethyl sulfate (4.16 g, 33 mmol). After the solution was left for 1 hour at room temperatures, terephthalic aldehyde (13.4 g, 100 mmol) was added to the solution and dissolved therein by heating. Subsequently, piperidine (0.47 ml) was added, and refluxed for 5 hours. A separated substance was removed by heating filtration. A hot filtrate was mixed with a mixed solvent of ethanol (50 ml) and acetone (16.7 ml), which was left overnight at room temperature. A yellow separated substance was batched off by filtration, which was washed with ethanol and acetone, and then dried under reduced pressure to obtain FPP. Its yield was 4.81 g (46%) and its melting point was from 210 to 213°C.

The derivative wherein lactose was introduced into chitosan as described in the foregoing Synthesis Example 1 (CH-LA) (1g) was dissolved in distilled water (100 ml), to which the FPP (368 mg, 1.15 mmol) obtained by the foregoing was added, and the resultant was stirred for 30 minutes. The pH was adjusted to 4.75 by 1N NaOH, and an injection solvent (8 ml) of cyano sodium borohydride (112 mg, 1.73 mmol) was added. The resultant was stirred for 24 hours at room temperature in the state of light shielding, which was poured into acetone (360 ml) to obtain a precipitate. The precipitate was sufficiently washed with methanol and acetone, and dried under reduced pressure to obtain VL-RC. Its yield was 728 mg and its substitution degree was 1.55%.

An aqueous solution containing 0.01 wt% of CH-LA, an aqueous solution containing 0.001 wt% of FPP, and an aqueous solution of 0.01 wt% of VL-RC obtained in Synthesis Example 2 were prepared. Absorption characteristics of the respective aqueous solutions were measured by a fluorescence spectroscopy. A part of these results are shown in FIG. 1 by comparison.

As shown in spectrums shown in FIG. 1, regarding VL-RC, the maximum absorption was shown in the vicinity of from 340 to 350 run as pure FPP, and it was confirmed that visible light reactive FPP was introduced. Further, when the chitosan material derived from shrimp shell and the chitosan material derived from cuttlefish cartilage were used, the derivatives showing similar absorption in the visible regions could be synthesized as well.

### (Example 1)

2% water solutions of UV-RC and VL-RC obtained in the foregoing Synthesis Examples 1 and 2 were prepared. The respective aqueous solutions were irradiated with light having various wavelengths by an irradiation device made by Ushio, Inc., and a strength test of adhesiveness of the crosslinked chitosan matrix was conducted by the procedure described in Example 4 of WO00/27889.

More specifically, 2 slices of edible ham being 2 mm thick cut in a size of 2x3 cm were arranged to obtain a size of 2x6 cm. The foregoing respective solutions were applied thereto so that an application size became 2x2 cm and 2 mm thick with a central focus on the interface between the two slices of ham.

Immediately after that, the applied solutions were illuminated with light for 30 sec to bond the two slices of ham. One of the bonded two slices of ham was fixed to a stand by a clip, an end of the other slice of ham was progressively weighted, and the weight was measured at which the bonded two slices of ham split. Evaluation was made by a weight per cross-sectional area of gel (10² kg/m²) when the crosslinking chitosan gel bonding the ham was split. The results of the respective derivatives were relatively shown based on 3x10² kg/m² when UV-RC (crab) was split (Table 1).

**Table 1**

| Material | Wavelength of irradiation light (nm) | | |
|---|---|---|---|
| | 245 | 365 | 400-500 |
| UV-RC (crab) | ++ | +++ | - |
| UV-RC (shrimp) | ++ | +++ | - |
| UV-RC (cuttlefish) | +++ | +++ | - |
| VL-RC (crab) | + | + | +++ |
| VL-RC (shrimp) | + | + | +++ |
| VL-RC (cuttlefish) | + | ++ | +++ |
| (-): not insolubilized | | | |

### (Example 2) Holding characteristics of high molecular substance

Holding characteristics of a polymer material (protein) was examined by using prepared UV-RC derived from crab. In order to change solubility and holding characteristics of the polymer substance, UV-RC wherein an introduction ratio of lactose was 0, 2, 5, 10, or 20% was prepared. 1 wt% solution of each UV-RC and 10 wt% bovine serum albumin solution were mixed at a ratio of 4:1 to adjust pH to 6.0 or 7.0. Then, each amount of an insoluble matter generated in the mixed solution and each viscosity of the mixed solution were compared. The viscosity of the mixed solution was estimated from a migration rate of the mixed solution in a glass tube. The results are shown in the following Table 2.

**Table 2**

| Lactose introduction ratio of UV-RC (%) | pH | Generation degree of insoluble matter (-: not generated) | | Viscosity increase due to adding albumin (+: increased) |
|---|---|---|---|---|
| | | Without albumin | Albumin added | |
| 0 | 6 | + | + | + |
| | 7 | ++++ | +++++ | + |
| 2 | 6 | - | - | + |
| | 7 | ++++ | +++++ | + |
| 5 | 6 | - | - | - |
| | 7 | ++ | +++ | + |
| 10 | 6 | - | - | - |
| | 7 | + | ++ | - |
| 20 | 6 | - | - | - |
| | 7 | - | ± | - |

As shown above, when lactose was not introduced, the insoluble matter was generated in any pH. Meanwhile, by introducing lactose, solubilization under the physiological conditions (pH 6 and/or 7) could be achieved. Further, it was found that when the polymer substances such as protein (albumin) was taken in, the higher the lactose introduction ratio was, the harder the insoluble deposit was to generate, that is, the case of the higher lactose introduction ratio is advantageous as a photo-crosslinking chitosan derivative holding a drug or the like. Further, it was shown that when the lactose introduction ratio was about 5% or more, preferably about 10% or more under the physiologic conditions or in the vicinity thereof (pH 6 or 7), lots of polymer substances such as protein can be suitably held.

### (Example 3)

### Drug release from crosslinked chitosan matrix (1)

An aqueous solution prepared by dissolving 1 mg/ml of trypan blue (model for an acid with low molecular weight) or toluidine blue (model for a base with low molecular weight) in a phosphate buffer (PBS) and 2 wt% aqueous solution of UV-RC synthesized from crab-derived chitosan were mixed at a ratio of 1:3. 50 µl of the prepared mixed solution was added to polyethylene 24-wells tissue culturing multiwell, which was irradiated with UV to form a crosslinked chitosan matrix layer containing low molecular weight coloring matter on the bottom face of the well. This matrix was washed with PBS, to which 1 ml of PBS was added. The resultant was mildly rotated by a rotary shaker at room temperature and incubated. PBS was changed every day

At intervals of given time, PBS was retrieved, and the amount of the coloring matter eluted into PBS was estimated by absorbance (OD₆₄₀). Time change of residual coloring matter in the crosslinked chitosan matrix is shown in FIG. 2.

As shown in FIG. 2, the low molecular weight coloring matter held in the crosslinked chitosan matrix (UV-RC) having a basic amino group was totally held in a gel over a long period due to ionic interaction in the case of the acidic trypan blue. However, in the case of the basic toluidine blue, almost all the low molecular weight coloring matter was eluted in PBS within a day

### (Example 4)

### Drug release from crosslinked chitosan matrix (2)

A test was conducted under the same conditions as in Example 3, except that the low molecular weight coloring matter was changed to the polysaccharide, heparin, and the proteins, albumin and protamine, and respective elution characteristics of polymer substances were compared. The amount of eluted heparin was measured by using carbazole assay (Guo, Y., Conrad and H. E., Anal. Biochem., 176, 96-104 (1989), and the amount of protein was measured by using protein assay kits. The results are shown in FIG. 3.

As shown in FIG. 3, heparin and the like, being polymer substances, were effectively captured in the crosslinked chitosan matrix, and elution thereof was controlled over a long period. Heparin, being an acidic polysaccharide, had the highest holding characteristics. Regarding the basic protein, about 30% thereof was eluted in a first day, and then elution was reduced. The neutral protein, albumin, showed behavior intermediate to both. In any case, significant elution of the held polymer substance was not shown on and after the second day. Therefore, it was found that the crosslinked chitosan matrix rigidly holds polymer substances such as polysaccharides and proteins over a long period regardless of characteristics such as acidity, neutrality, and alkalinity.

### (Example 5)

### Drug release from crosslinked chitosan matrix (3)

An elution test was conducted as in Examples 3 and 4, except that the held substance was changed to growth factors, FGF-1, FGF-2, heparin bonding EGF (HB-EGF), and VEGF₁₆₅. Elution amounts of the respective growth factors were estimated by ELISA method (Sigma Aldrich) by using commercially available heparinated beads.

In result, elution profiles almost corresponding with those of albumin in Example 4 were observed for all the growth factors. Of the respective growth factors, slight differences were shown in terms of initial elusion on the first day. FGF-1 showed the least elusion amount. The elusion amount was increased in the order of FGF-2, HB-EGF, and VEGF₁₆₅. However, significant differences among these elution amounts were not shown. In any growth factor, the initial elution amount on the first day was not beyond 20%. As a representative example, the result in the case that FGF-2 was held is shown in FIG. 4.

### (Example 6)

### Drug release from crosslinked chitosan matrix (4)

A test similar to that in Example 4 was conducted by using albumin as a drug to be held and changing concentration of UV-RC in the range from 0.5 to 1.5 wt%. FIG. 5 shows the result that the albumin amount held in the crosslinked chitosan matrix after the lapse of the first day is plotted in relation to the UV-RC concentration. As shown in FIG. 5, it is evident that the initial elusion characteristics of the drug, particularly polymer substances, depends on the content of UV-RC, that is, the molecule density of chitosan.

From the results of Examples 3 to 6, it was demonstrated that polymer substances such as polysaccharides, protein, and growth factors were effectively held in the crosslinked chitosan matrix of the present invention. In particular, it was demonstrated that elusion on and after the second day was almost totally inhibited, and stable holding was attained. Further, differently from low molecular substances, these high molecular substances were well held regardless of molecule characteristics such as acidity, neutrality, and basicity. Particularly, it was shown that drugs having lots of acid groups had high holding stability. Further, in the foregoing proteins and cell growth factors, their elusion inhibitory effects were maintained over 7 days or more. Meanwhile, when PBS was substituted with PBS to which chitinase/chitosanase mixed enzymes which decompose chitosan hydrogel was added on the seventh day, elusion of polymer substances was gradually started. Such sustained-release characteristics due to biodegradation of the chitosan matrix continued at least 1 week.

### (Example 7)

### Culture of HUVEC on crosslinked chitosan matrix

Crosslinked chitosan matrices holding the growth factors (FGF-1, FGF-2, HB-EGF, and VEGF₁₆₅) prepared in Example 4 were formed on a cell culture dish. The matrices were washed with PBS for 1, 3, and 7 days. After that, HUVEC was seeded and cultured for 3 days. After that, growth characteristics on the matrices holding the respective growth factors were evaluated. Further, similar evaluation was made for a culture medium in which chitinase/chitosanase enzymes were added to a dish containing a matrix washed for 7 days. The results are shown in Table 3. The numerical values in the table are relative values where the growth characteristic in the case where culture was made on a matrix having only UV-RC (no growth factor) was set to 100.

**Table 3**

| | Washing days of UV-RC (day(s)) | | | | |
|---|---|---|---|---|---|
| | 0 | 1 | 3 | 7 | 7+Enzyme |
| FGF-1 | 132±14 | 126±19 | 104±18 | 100±19 | 129±14 |
| FGF-2 | 173±27 | 142±11 | 104±12 | 97±17 | 178±27 |
| VEGF₁₆₅ | 159±14 | 151±17 | 115±20 | 107±13 | 173±21 |
| HB-EGF | 109±12 | 104±14 | 108±17 | 104±21 | 112±17 |

Washing day 0 means that HUVEC was seeded without washing the crosslinked chitosan matrix. As evidenced by Table 3, in the case of non-washing in which there was the initial release of the growth factor from the matrix, the highest cell growth characteristics were shown. When release of the growth factor was almost totally inhibited after washing one or more days, there were some cases wherein only growth characteristics equal to that of the matrix containing no growth factor (numerical value: 100) were seen. However, when the enzymes were added, by re-releasing the respective growth factors associated with decomposition of the crosslinked chitosan matrix, growth characteristics equal to of the non-washed matrix was recovered. As a representative example, the result of the case wherein FGF-2 was held is shown in FIG. 6. The vertical axis of the graph of FIG. 6 represents relative values of growth characteristics (growth rate), and correlates with the number of growth cells. A dotted line in FIG. 6 represents a base line of the numerical value 100.

### (Example 8)

### In vivo release of trypan blue from crosslinked chitosan matrix

In accordance with Example 3, a crosslinked chitosan matrix holding trypan blue was prepared. 100 µl of this crosslinked matrix was buried in a position being 2 cm apart from a home of a tail on the back of a mouse. After the lapse of a given period of time, the matrix was taken out and washed. Chitosan was decomposed by 100 mM of sodium nitrate, and residual amounts of trypan blue were measured. The result is shown in FIG. 7.

In the result of Example 3, trypan blue was hardly released into PBS from the crosslinked chitosan matrix (FIG. 2). Meanwhile, in the case of in *vivo* release, as shown in FIG. 7, the chitosan matrix was decomposed by enzymes existing in the body, trypan blue was gradually released over about two weeks, and the amount of the residual coloring matter in the matrix was decreased.

### (Example 9)

### In vivo release of growth factor from crosslinked chitosan matrix

In accordance with Example 5, a composition containing FGF-1 or FGF-2 was prepared, and each 100 µm thereof was irradiated with UV to obtain the crosslinked chitosan matrix. Further, the crosslinked chitosan matrix in which heparin having a final concentration of 20 µg/ml coexisted in order to stabilize the growth factor was also prepared. These crosslinked chitosan matrices were buried into the back of a mouse similarly to in Example 8 and left for 4 days. After that, 1x1 cm of skin around the buried matrix was peeled, skin tissues were finely cut, and red cell hemoglobin in the tissues was extracted by a hemolytic agent (Sigma Aldrich). From the amount of hemoglobin detected by a hemoglobin assay kit (Sigma Aldrich), blood capillary derivation characteristics in the region was estimated. The results are shown in FIG. 8. Amounts of the growth factor added to the composition were changed as 1, 4, and 15 µg/100 µg.

As shown in FIG. 8, it was found that by making 4 µg or more of FGF be held in the crosslinked chitosan matrix, hemoglobin (Hb) associated with angiogenesis in the vicinity of the region in which the gel was buried was significantly increased. Such effects were further intensified by adding heparin which could be expected to provide the effect to maintain the growth factor by ionic interaction and to improve the function of FGF.

As a control, burying a chitosan derivative which contains the growth factor but is not provided with photo-crosslinked was conducted concurrently. However, in this case, increase of hemoglobin was not found. It can be considered that the reason thereof was the growth factor was diffused and released in a short time, and therefore, sustained-release effects could not be obtained. Further, when only heparin was held in the crosslinked chitosan matrix, Hb amount was the same degree as in the case that FGF was not added.

Further, FGF concentration was fixed to 4 µg, and a similar test was continued for 15 days. The increase trend of hemoglobin was maintained. As shown in FIG. 9(B), particularly when FGF-2 was used, maintenance effects of Hb increase trend was significant. After 15 days, 1 mg or more level was maintained. Signs of canceration were never observed in the vicinity of the buried part.

### (Example 10)

### Healing acceleration effects in wound model

### (1) Formation of skin wound model in mouse

In this experiment, C57BL/ksj db/db, which was a female diabetes variant mouse and a normal mouse of its littermate (db/+) (CREA Japan Co.) were used. All mice were provided with standard experimental diet and water, and used for the experiment when they became over ten weeks old. Before the experiment, urine of the mice was checked with respect to glucose and protein by using a test specimen (Uro-Labstrix: Bayer Medical Co.). In result, it was diagnosed that the db/db mouse had severe diabetes, and db/+ mouse was normal.

Each mouse was given anesthetic by diethyl ether. After back hair was completely shaven, a circular wound (about 100 mm²) over a full skin thickness was formed on the upper part of the back of each mouse by using edged scissors and a surgical knife.

### (2) Wound healing acceleration effects

100 µl of a composition in which human recombinant FGF-2 (Pepro Tech EC Co.) was added to 20 mg/ml of UV-RC solution, or a composition in which FGF-2 was not added was applied to the wound of each mouse. The wound was irradiated with ultraviolet rays being 2 cm apart from the wound for 20 sec to obtain the crosslinked chitosan matrix. A mouse in which a similar wound (about 100 mm²) was formed to which no treatment was conducted was used as a control. Change of the wound area of each mouse was measured every two days by using a caliper rule. According to visual observation, the crosslinked chitosan matrix became a hydrogel hydrated through the healing process.

Regarding the db/db mouse, photographs of the wound on the second, fourth, eighth, and 14th days from forming the wound are shown in FIG. 10. Further, wound occlusion rates were evaluated as a function between ratio (%) of non-occlusion area in relation to the area in forming the wound and time. The result is plotted in FIG. 11.

As evidenced by FIGs. 10 and 11, in the control wound (A), only about 50% of the wound area was occluded even on the 14th day. It was on and after 20th day when about 80% of wound occlusion was attained. Meanwhile, the area of the wound covered by the crosslinked chitosan matrix holding FGF-2 (C) shrunk very rapidly. About on the sixth to tenth day, about 80% of the wound was occluded, and on the 14th day, the wound was almost completely healed. Further, the wound covered by only the crosslinked chitosan matrix (not containing FGF-2) (B) showed a result intermediate to both. It was confirmed that the healing effects by the composition containing the photo-crosslinkable chitosan derivative of the present invention were, in particular, significantly demonstrated during the initial 2 days, and such effects were continued until healing was attained.

Meanwhile, regarding the db/+mouse, photographs of the wound on the second, fourth, and tenth days from forming the wound are shown in FIG. 12. The graph of wound healing rates is shown in FIG. 13.

In the case of the normal db/+ mouse, wound healing was faster than in the db/db mouse having a healing disorder under all conditions. However, when covered with the crosslinked chitosan matrix of the present invention, healing was significantly accelerated compared to the untreated control. More specifically, in the control (A), about 70% of wound occlusion was shown on the tenth day. Meanwhile, in the case of covering with the crosslinked chitosan matrix ((B) and (C)), about 80% of wound occlusion was attained within eight days. Further, healing acceleration effects by adding FGF-2 was significant in the first stage of the healing process (within about six days). After that, no significant difference due to the adding of FDF-2 was seen. It can be considered that the reason thereof was in the normal db/+ mouse, growth factors and the like required for healing were provided *in vivo* as well. That is, the crosslinked chitosan matrix of the present invention capable of sustained-releasing a growth factor such as FGF-2 appropriately and over a long period of time is particularly suitable for treatment of intractable wounds such as diabetic skin ulcers.

Healing promotion effects equal to or more than the foregoing could be found when heparin (swine small bowel-derived, Scientific Protein Laboratories Co.) was further added.

### (Example 11)

### Histological observation of wound model in db/db mouse

Part of skin containing wound tissues was taken on each measurement day in Example 10. The taken sample was fixed in 10% formaldehyde solution, embedded in paraffin, and sliced in 4 µm thick in a section perpendicular to the wound surface (Yamato Kohki Co.). The sliced piece was put on a glass slide, and stained with hemotoxylin-eosin (HE) reagent.

FIG. 14 shows photographs showing each piece on the second, fourth, eighth, and 16th day. Arrows shown in the figure indicate edges of epithelialized tissues. In the untreated control (A), epithelialization did not proceed even on the 16th day However, in the wound covered with the chitosan matrix holding FGF-2 (C), epithelialization rapidly proceeded, and almost complete epithelialization was attained on the 16th day. In the chitosan matrix containing no FGF-2 (B), epithelialization corresponding to medium between the both was observed.

Photomicrographs of the pieces on the fourth day from forming the wound are shown in FIG. 15. The symbol V affixed in the figure indicates the edge of formed granulation tissues, the white arrow indicates the angiogenesis region, and the black arrow indicates the remaining chitosan matrix. In the wound covered with the chitosan matrix holding FGF-2 (C), angiogenesis and granulation of tissues proceeded. In the case of covering with the chitosan matrix containing no FGF-2 (B), slight angiogenesis was also observed. However, in the untreated control (A), any of angiogenesis and granulation could not be observed.

For example, there is a report that when an aqueous solution of FGF-2 was added to a wound opening of the db/db mouse once a day, only slight effects could be shown in terms of acceleration of re-epithelialization (Tsuboi, R. and Rifkin, D.B., J. Exp. Med., 1990: 172, 245-251). The inventors thereof and the like infer that when the aqueous solution of FGF-2 was used, a large wound cap was formed at the wound opening, which adversely affected ambulato of keratinocyte. According to the present invention, the wound opening was covered with chitosan matrix (hydrogel). Therefore, it can be considered that a large wound cap was not formed, and FGF-2 in the matrix accelerated re-epithelialization.

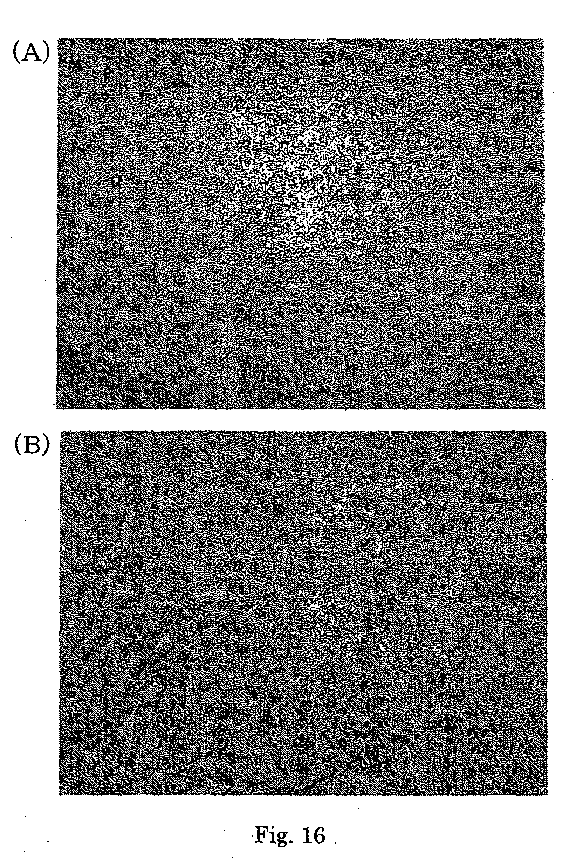

Further, FIG. 16 shows photomicrographs of the samples wherein the piece on the fourth day from forming the wound was provided with immune staining with CD34. It was observed that compared to the untreated sample (A), in the sample covered with the chitosan matrix holding FGF-2, vascularization was significantly increased. The results of average numbers of blood vessels existing per one visual field of a microscope are shown in the following Table 4.

**Table 4**

| Day | Fourth day | 16th day |
|---|---|---|
| Control | 18.2 | 33.4 |
| Chitosan matrix | 15.1 | 31.5 |
| FGF-2/chitosan matrix | 37.4 | 41.6 |

### (Example 12)

The abdomen of a rat was cut, and an ulcer model was formed on the stomach wall thereof with a laser knife. Healing acceleration effects similar to the foregoing was confirmed by pathological tissue observation. More specifically, the healing rate was improved in order of the crosslinked chitosan matrix (without GF) and the crosslinked chitosan matrix+GF. This suggests that even in the nonphysiological environment by stomach acid (about pH2), chitosan gel protected drugs such as GF to some extent, and contributed to healing. Ultimately, in a plurality of animal experiments, healing was confirmed in up to about a half period compared to the case without covering with the crosslinked chitosan matrix. However, practically, when used on the stomach wall which is under a low pH environment due to stomach acid, it is preferable that application is made under coexistence with substances inhibiting action and secretion of stomach acid such as a buffer and H2 blocker. However, in digestive organs on and after the intestine, GF and the like can be well protected and superior wound healing acceleration effects can be demonstrated without coexistence with such substances.

The foregoing effects were shown not only in using the ultraviolet crosslinking type (UV-RC), but also in using the visible light crosslinking type VL-RC similarly.

### INDUSTRIAL APPLICABILITY

The crosslinking chitosan derivative used in the present invention has both a lactose part and a photoreactive group. Therefore, the crosslinking chitosan derivative is soluble in water in physiological pH in the vicinity of neutrality. By irradiating an aqueous solution thereof with light (UV or visible light), the aqueous solution becomes an insoluble hydrogel having a strength equal to that of soft rubber generally within 10 sec. The formed crosslinked chitosan matrix occludes wounds well, and protects and shrinks the wound in an appropriate humidified healing environment.

Further, the crosslinked chitosan matrix of the present invention can hold particularly well polymer substances such as polysaccharides, proteins, and growth factors. Along with biodegradation of the matrix *in vivo,* the crosslinked chitosan matrix has an ability to sustained-release the held substance. In particular, when a wound is covered with the crosslinked chitosan matrix holding a therapeutic agent for wounds containing a high diffusive growth factor such as FGF, wound healing is accelerated by appropriately sustained-releasing the growth factor or the like, and canceration is not induced. Therefore, the composition of the present invention is particularly useful for promoting the healing of intractable wounds in which tissues become necrotic such as diabetic skin ulcers.

The crosslinking chitosan derivative of the present invention can be used as a carrier for holding and sustained-releasing a growth factor relating to wound healing other than FGF such as platelet-derived growth factor (PDGF), transforming growth factor-β (TGF-β), vascular endothelial cell growth factor (VFGF), and epidermal growth factor (EGF).

Further, the crosslinked chitosan matrix of the present invention can be used as a culture medium material for cell culture or tissue regeneration.

## Claims

1. Medical composition containing a photo-crosslinkable chitosan derivative and a wound healing promoter.

2. Composition according to Claim 1, **characterized in that** the photo-crosslinkable chitosan derivative is a chitin/chitosan comprising units represented by the following formula (I): 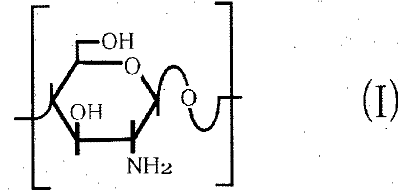 which is formed by incorporating a carbohydrate chain containing a reducing terminal to at least one part of amino groups of the glucosamine units and incorporating a photoreactive group to at least another part of amino groups pf the glucosamine units.

3. Composition according to Claim 1, **characterized in that** it contains at least 3 mg/ml of the photo-crosslinkable chitosan derivative.

4. Composition according to Claim 1, **characterized in that** the wound healing promoter is a growth factor.

5. Composition according to Claim 4, **characterized in that** the growth factor is FGF-1, FGF-2, HB-EGF, VEGF₁₆₅ or HGF.

6. Composition according to Claim 1, **characterized in that** it further contains glycosaminoglycans.

7. Composition according to Claim 6, **characterized in that** the glucosaminoglycans are heparin or heparan sulphate.

8. Crosslinked chitosan derivative matrix obtainable by irradiating light to the composition according to any one of Claims 1 to 7 and carrying a wound healing promoter and optionally glycosaminoglycans.

9. Crosslinked chitosan derivative matrix according to Claim 8, **characterized in that** its crosslinking degree by the photoreactive groups is 30 to 100%.

10. Crosslinked chitosan derivative matrix according to Claim 8 or 9, **characterized in that** the wound healing promoter is a growth factor.

11. Drug-sustained-release body composed of the crosslinked chitosan derivative matrix according to any one of Claims 8 to 10.

12. Base material for tissue regeneration or cell culture comprising the crosslinked chitosan derivative matrix according to any one of Claims 8 to 10.

13. Wound dressing for treatment of an intractable skin disease comprising the composition according to any one of Claims 1 to 7.
